**Europäisches Patentamt**

**European Patent Office**

**Office européen des brevets**

(11) Publication number : **0 396 422 B1**

# EUROPEAN PATENT SPECIFICATION

(45) Date of publication of patent specification :
02.03.94 Bulletin 94/09

(51) Int. Cl.⁵ : **A61K 7/48, A61K 7/42**

(21) Application number : **90304849.4**

(22) Date of filing : **04.05.90**

(54) **Skin composition.**

The file contains technical information
submitted after the application was filed and
not included in this specification

(30) Priority : **05.05.89 GB 8910366**

(43) Date of publication of application :
**07.11.90 Bulletin 90/45**

(45) Publication of the grant of the patent :
**02.03.94 Bulletin 94/09**

(84) Designated Contracting States :
**AT BE CH DE DK ES FR GB GR IT LI NL SE**

(56) References cited :
**FR-A- 2 440 933
GB-A- 1 370 236
US-A- 4 096 240
Manufacturing Chemist vol. 58, no. 11, Novem-
ber 1987, London,GB pages 62 - 63; Philip
Alexander: "Out in the Sun"**

(73) Proprietor : **UNILEVER PLC
Unilever House Blackfriars P.O. Box 68
London EC4P 4BQ (GB)**

(84) **GB**
Proprietor : **UNILEVER N.V.
Weena 455
NL-3013 AL Rotterdam (NL)**

(84) **BE CH DE DK ES FR GR IT LI NL SE AT**

(72) Inventor : **Governor, Rusi
2B-Anusandhan, Chakala
Andheri (East), Bombay 400 099 (IN)**
Inventor : **Natraj, Collur Visweswariah
3B-Anusandhan, Chakala
Andheri (East), Bombay 400 099 (IN)**

(74) Representative : **Ford, Michael Frederick et al
MEWBURN ELLIS 2 Cursitor Street
London EC4A 1BQ (GB)**

Jouve, 18, rue Saint-Denis, 75001 PARIS

## Description

The present invention relates to skin cosmetic compositions and more particularly to skin cosmetic compositions having the effect of lightening the skin.

In GB 1 370 236 there is described a composition intended for application to the skin which contains niacin as a skin lightening agent. The method by which niacin acts to effect skin lightening is postulated as being a retardation of melanin dispersion or distribution into the epidermis.

It was later disclosed in GB 1 533 119 that niacinamide is useful as a skin lightening agent and does not cause the skin flushing reaction due to vasodilation of the blood vessels which is often associated with niacin.

Skin compositions have also been formulated for the purpose of maintaining the colour of skin against darkening following exposure to ultra-violet light. These compositions have generally been based on materials which deflect and scatter incident ultra-violet light of the wavelength which produces burning and tanning of the skin, or which absorbs this light.

The UV range is divided into three regions; UV - A, having a wavelength of from about 320 to 400 nm which gives a tanning effect without inflammation; UV - B, having a wavelength of from about 290 to 320 nm which is responsible for erythema (sunburn) and eventually for tanning; and UV-C, having a wavelength of from about 200 to 290 nm, normally absorbed by the ozone layer, but which is potentially very damaging to the skin.

It will be appreciated that ethythema, melanogenesis and pigmentation of the skin are closely related responses to irradiation by UV light. The photobiological changes that cause erythema also lead to melanogenesis and increased pigmentation. Hence exposure to sunlight leads to darkening of the skin by immediate pigment darkening of already formed melanin, and also by formation of new melanin which subsequently darkens.

The invention is therefore concerned with providing a composition for topical application to the skin which not only lightens the skin but which also protects against immediate pigment darkening and melanogenesis due to exposure to solar radiation.

To prevent darkening of existing melanin, the skin has to be protected broadly across the UV-A range of from about 320 to 400 nm. We have found that by careful selection of sunscreens we can protect the skin against the darkening effects of radiation across almost the whole UV-A range.

In GB 1 533 119 referred to above, there are disclosed skin compositions comprising niacinamide and a sunburn or suntan protective amount of one or more ultraviolet absorbing sunscreens. The sunscreen combination actually disclosed is 2-hydroxy -4-octyloxybenzophenone and 2-ethoxyethyl -p-methoxy-cinnamate which does not afford protection up to the maximum of the UV-A range.

We have also found that it is advantageous for the coverage given by skin creams and lotions etc. to be even and uniform. If there are gaps in the coverage then UV-A and UV-B light will penetrate the skin giving some darkening and melanogenesis.

## BRIEF SUMMARY OF THE INVENTION

It has now been found that a cosmetic composition giving improved skin lightening and which is easily spread on the skin can be obtained.

Accordingly the invention provides a cosmetic composition for lightening skin which composition comprises
(a) from 0.1 to 10% by weight of niacinamide or a precursor thereof;
(b) from 0.1 to 10% by weight of a sunscreen mixture comprising 4-tertiary butyl-4'-methoxy dibenzolymethane and 2-ethyl hexyl methoxy cinamate; and
(c) from 0.1 to 5% by weight of a silicone compound.

## DETAILED DESCRIPTION OF THE INVENTION

### (a) Niacinamide

The compositions of the invention comprise from 0.1 to 10%, preferably from 0.5 to 5% by weight of niacinamide or a precursor thereof.

Niacinamide is the amide of niacin and is also known as nicotinamide or pyridine-3-carboxylic acid. An example of a compound which is a niacinamide precursor is niacinamide ascorbate.

In the following description the term "niacinamide" will be used to include both niacinamide itself or a precursor thereof which is capable of releasing niacinamide on the skin, unless otherwise stated.

Niacinamide is an effective skin lightener and is free from the skin irritancy and flushing sensation sometimes observed with niacin.

(b) Sunscreen

The compositions of the invention also comprise from 0.1 to 10% by weight, preferably from 0.1 to 5% of a sunscreen mixture. The sunscreen mixture comprises 4-tertiary butyl-4'-methoxy dibenzoylmethane, available under the trade name PARSOL 1789 from Givaudan, and 2-ethyl hexyl methoxy cinnamate, available under the trade name PARSOL MCX from Givaudan.

The sunscreen mixture employed in the compositions of the invention preferably comprises from 0.01 to 4% by weight of 4-tertiary butyl-4'-methoxydibenzoylmethane together with from 0.1 to 4% by weight of 2-ethyl hexyl methoxycinnamate.

PARSOL 1789 shows absorbance across the entire UVA region, in contrast to the sunscreen material used in GB 1 533 119, CYASORB UV 531, which shows low absorbance towards the upper end of the UV-A region.

PARSOL 1789 therefore has a superior sunscreen effect to CYASORB UV 531 in that it has effect over the whole tanning range of the spectrum.

(c) Silicone compound

The composition of the invention further comprises from 0.1 to 5% by weight of a silicone compound, which is preferably dimethyl polysiloxane.

The silicone compound gives the composition greater spreadability and the skin lightening and sunscreen effects are thereby distributed evenly over the surface of the skin. The compound also imparts an improved smoothness and softness to the skin which is pleasing to the consumer.

PRODUCT FORM AND USE

The composition according to the invention can be formulated, together with a cosmetically acceptable vehicle conventional in the art and other ingredients as necessary, to provide a product which is suitable for topical application to the skin. Examples of product forms include creams, lotions, ointments and propellant driven or pump spray preparations.

In use, the composition can be applied topically to the skin, especially those areas which are or may become exposed to sunlight. Conveniently, from 1 to 5 ml of the composition can be spread over the skin surface using the fingers or a suitable applicator, once or twice daily. The composition can also be applied to the skin at night, for example as a nightcream.

The invention is further illustrated by reference to the following example of a cosmetic cream.

|  | % weight |
|---|---|
| Stearic acid | 16.0 |
| PARSOL 1789 | 0.5 |
| PARSOL MCX | 1.2 |
| Dimethyl polysiloxane | 0.2 |
| Niacinamide | 3.2 |
| Isopropyl palmitate | 4.4 |
| Methyl/propyl parabens | 0.15 |
| Perfume | 0.2 |
| Water | to 100 |

The above cream gives a gradual and reversible skin lightening effect. The colour of the skin returns to normal within a few weeks of discontinuing use of the cream.

COMPARATIVE EXAMPLE

The cream of the Example set out above (A) was compared with the cream of the Example of GB 1 533 119 (B), which had the following formulation:

|  | % weight |
|---|---|
| Stearic acid | 15.0 |
| Cetyl alcohol | 0.5 |
| CYASORB UV 531 | 0.4 |
| GIVTAN F[1] | 1.0 |
| Glycerol | 1.0 |
| Niacinamide | 3.0 |
| Isopropyl myristate | 4.0 |
| Glyceryl monostearate | 1.0 |
| Caustic Potash (17.8%) | 2.8 |
| Formalin | 0.05 |
| Perfume | 0.2 |
| Water | to 100 |

1 - GIVTAN F is 2-ethoxyethyl-p-methocycinnamate.

A consumer panel was set up to subjectively evaluate A and B, and the responses of panel members to a number of questions were recorded. The results were as follows:

|  | A | B |
|---|---|---|
| Actually makes skin fairer | 3.90 | 3.65 |
| Only cream that makes fairer | 2.91 | 2.65 |
| Works from within to make fairer | 3.66 | 3.41 |
| Prevents tanning | 4.08 | 3.78 |
| Good make up base | 3.94 | 3.53 |
| Makes skin soft and smooth | 4.28 | 4.01 |
| Safe to use | 4.56 | 4.15 |
| Works gently/mildly | 4.26 | 4.05 |

The results of the comparison show that the composition of the invention is seen by consumers to be more effective in skin lightening performance in all respects than the cream according to GB 1 533 119. It is also seen to give an improved softness and smoothness to the skin and to be mild in use.

**Claims**

1. A cosmetic composition for lightening skin comprising
   (a) from 0.1 to 10% by weight of niacinamide or a precursor thereof;
   (b) from 0.1 to 10% by weight of a sunscreen mixture comprising 4-tertiary butyl-4'-methoxy dibenzoylmethane and 2-ethyl hexyl methoxy cinnamate; and
   (c) from 0.1 to 5% by weight of a silicone compound.

2. A cosmetic composition according to claim 1 comprising from 0.5 to 5% by weight of niacinamide or a precursor thereof.

3. A cosmetic composition according to any preceding claim, wherein the niacinamide precursor is niacinamide ascorbate.

4. A cosmetic composition according to any preceding claim, comprising from 0.1 to 5% by weight of the sunscreen mixture.

5. A cosmetic composition according to claim 4, comprising from 0.01 to 4% by weight 4-tertiary butyl-4'-methoxy-dibenzoylmethane and from 0.1 to 4% by weight 2-ethyl hexyl methoxycinnamate.

6. A cosmetic composition according to claim 1, wherein the silicone compound is dimethyl polysiloxane.

## Patentansprüche

1. Eine kosmetische Zusammensetzung zum Aufhellen der Haut, die umfaßt
   (a) 0.1 bis 10 Gew.-% Niacinamid oder eines Vorläufers davon
   (b) 0.1 bis 10 Gew.-% einer Sonnenschutzmittelmischung aus % 4-tertiär-Butyl-4'-Methoxydibenzoylmethan und 2-Ethylhexylmethoxycinnamat; und
   (c) 0.1 bis 5 Gew.-% einer Silikonverbindung.

2. Eine kosmetische Zusammensetzung nach Anspruch 1, die 0.5 bis 5 Gew.-% Niacinamid oder eines Vorläufers davon umfaßt.

3. Eine kosmetische Zusammensetzung nach einem vorhergehenden Anspruch, wobei der Niacinamidvorläufer Niacinamidascorbat ist.

4. Eine kosmetische Zusammensetzung nach einem vorhergehenden Anspruch, die 0.1 bis 5 Gew.-% der Sonnenschutzmittelmischung umfaßt.

5. Eine kosmetische Zusammensetzung nach Anspruch 4, die 0.01 bis 4 Gew.-% 4-tertiär-Butyl-4'-methoxydibenzoylmethan und 0.1 bis 4 Gew -% 2-Ethylhexylmethoxycinnamat umfaßt.

6. Eine kosmetische Zusammensetzung nach Anspruch 1, wobei die Silikonverbindung Dimethylpolysiloxan ist.

## Revendications

1. Une composition cosmétique pour éclaircir la peau contenant:
   (a) de 0,1 à 10 % en masse de niacinamide ou d'un précurseur de celle-ci ;
   (b) de 0,1 à 10 % en masse d'un mélange d'écran solaire comprenant du 4-butyle tertiaire-4'-méthoxy dibenzoylméthane et du 2-éthyl hexyl méthoxy cinnamate; et
   (c) de 0,1 à 5 % en masse d'un composé silicone

2. Une composition cosmétique selon la revendication 1, comprenant de 0,5 à 5 % en masse de niacinamide ou d'un précurseur de celle-ci.

3. Une composition cosmétique selon l'une des revendications précédentes, dans laquelle le précurseur de niacinamide est de l'ascorbate de niacinamide.

4. Une composition cosmétique selon l'une des revendications précédentes, comprenant de 0,1 à 5 % en masse d'un mélange d'écran solaire.

5. Une composition cosmétique selon la revendication 4, comprenant de 0,01 à 4 % en masse de 4-butyle tertiaire-4'-méthoxydibenzoylméthane et de 0,1 à 4 % en masse de 2-éthyl hexyl méthoxycinnamate.

6. Une composition cosmétique selon la revendication 1, dans laquelle le composé silicone est du diméthyl

polysiloxane.